# EUROPEAN PATENT APPLICATION

(11) **EP 4 389 072 A1**
(43) Date of publication of application: **26.06.2024**
(21) Application number: 22888945.7
(22) Date of filing: 19.08.2022
(51) Int. Cl.: A61F 2/24

(54) **CATHETER ASSEMBLY, IMPLANT SYSTEM, IMPLANT DELIVERY SYSTEM, AND USE METHOD THEREFOR**

(30) Priority: 02.11.2021 CN 202111297260
(71) Applicant: Shanghai Trulive Medtech Co., Ltd, Shanghai 201206 (CN); Jiangsu Trulive Medtech Co., Ltd., Nantong, Jiangsu 226400 (CN)
(72) Inventor: ZHANG, Wei, Shanghai 201206 (CN); LI, Shaocun, Shanghai 201206 (CN); XU, Haoran, Shanghai 201206 (CN); WEI, Yongqiang, Shanghai 201206 (CN)
(74) Representative: Nordmeyer, Philipp Werner
(86) International application number: PCT/CN2022/113494
(87) International publication number: WO 2023/077912

(57) **Abstract**

A catheter assembly (1), an implant system, an implant delivery system and a method of use thereof. In the implant delivery system, an inner tube (120) is made of an elastic material, and an outer sheath tube (110) and a guidewire tube (130) are each made of a plastic material. A distal end portion of each of the outer sheath tube (110) and the guidewire tube (130) is shaped into a curved form to provide a curved channel for the inner tube (120). The inner tube (120) is elastically inserted through the curved channel. This dispenses with the need for bending control from an outer sheath tube control device (21) and an inner tube control device (22), reducing requirements and difficulties in designing them and thus resulting in cost savings. Moreover, it makes the implant delivery system more easily operable, safer and more reliable, with lower requirements on a surgical incision, and capable of delivering a stent through a curved access accurately to a designated site.

## Description

### TECHNICAL FIELD

The present invention relates to the field of medical devices and, in particular, to a catheter assembly, an implant system, an implant delivery system and a method therefor.

### BACKGROUND

Heart valves are openable and closeable flap-like structures in the organs of humans and some animals. The human heart has four valves: the aortic valve connecting the left ventricle to the aorta; the pulmonary valve connecting the right ventricle to the pulmonary artery; the mitral valve connecting the left atrium to the left ventricle; and the tricuspid valve connecting the right atrium to the right ventricle. All these valves function like one-way valves which allow blood flow in the forward direction but prevent its backward flow.

With the development of social economy and the aging of population, the incidence of valvular heart disease has increased significantly. Studies have shown that this figure has reached as high as 13.3% among those 75 years or older. At present, traditional surgical treatment remains the first choice for patients with severe valvular disease. However, for those with advanced ages, complications in multiple organs, a history of thoracotomy or poor heart functions, the traditional surgical approach is associated with high risk and high mortality or even precludes some patients.

Interventional valve implantation is a brand new minimally invasive valve replacement technique developed abroad in recent years, which involves loading valve prosthesis in a delivery system and delivering it into a human body through a catheter. The prosthesis can functionally replace the patient's dysfunctional native valve and improve his/her cardiac condition. This technique is able to treat valvular disease, without surgically opening the chest or stopping the heartbeat, which may cause significant trauma to the patient.

The structure of the human heart is very complex. In particular, the complex anatomy of the tricuspid valve, including the irregularly-shaped tricuspid annulus and the chordae tendineae and septomarginal trabecula in the ventricle, would interfere with interventional valve implantation. For tricuspid valve replacement through thoracotomy via an atrial appendage access, it is very difficult for a conventional straight-catheter delivery system to accurately deliver a prosthetic valve to a target site because a suitable incision for this purpose, which is desired to be aligned with the tricuspid annulus, must be made at a location close to the patient's back, placing extremely high requirements on the surgeon's skill. Usually, the actual incision is often largely offset from the tricuspid annulus, making it difficult to locate the target release site and to release the prosthetic valve at an appropriate location and angle. This may lead to incorrect operation of the operator, inaccurate stent placement, or even issues like severe paravalvular leakage or regurgitation in worse cases.

Further, conventional delivery systems with bending control typically include a handle bending control device including a pull thread and a pull ring, which can be manipulated to accomplish implant deployment and release, and catheter bending control. However, adding the handle bending control device can lead to increases in design and material cost and design complexity, and there is a risk of breakage between the pull thread and the handle, or between the pull thread and the pull ring, due to insufficient strength of the pull thread for the catheter bending control.

### SUMMARY OF THE INVENTION

It is an object of the present invention to a catheter assembly, an implant system, an implant delivery system and a method of use thereof, which can overcome problems with conventional delivery systems, including difficulties in delivery, inaccurate release, demanding requirements on the location of an incision, structural complexity, operation difficulties and insufficient safety.

To this end, the present invention provides a catheter assembly comprising an outer sheath tube, an inner tube and a guidewire tube that are sequentially sleeved one over another from the outside inwards. The inner tube is made of an elastic material, and each of the outer sheath tube and the guidewire tube is made of a plastic material, wherein a distal end of each of the outer sheath tube and the guidewire tube is shaped into a curved form to provide a curved channel for the inner tube, and wherein the inner tube is elastically inserted through the curved channel.

Optionally, the outer sheath tube may be made of a polymer material with a Shore D hardness of 70-90 HD.

Alternatively, the outer sheath tube may be made of a metal material with a Rockwell hardness of 20-50 HRC.

Optionally, the guidewire tube may be made of a polymer material with a Shore D hardness of 70-90 HD.

Alternatively, the guidewire tube may be made of a metal material with a Rockwell hardness of 20-50 HRC.

Additionally, the polymer material may comprise PA, PI or PEEK, and the metal material may comprise 304 stainless steel or 316 stainless steel.

Optionally, the inner tube may be made of Pebax, TPU, LDPE, NR or NBR, and may have a Shore D hardness of 40-70 HD.

Optionally, the outer sheath tube may comprise, disposed along an axis thereof, a first straight tube section and a first curved tube section, the first straight tube section located at a proximal end of the outer sheath tube, the first curved tube section located at a distal end of the outer sheath tube, a distal end of the first straight tube section detachably coupled to a proximal end of the first curved tube section.

Optionally, the guidewire tube may comprise, sequentially disposed along an axis thereof, a second straight tube section, a second curved tube section and a third straight tube section, the second straight tube section located at a proximal end of the guidewire tube, the third straight tube section located at a distal end of the guidewire tube, a distal end of the second straight tube section detachably coupled to a proximal end of the second curved tube section.

Optionally, the first straight tube section may be sleeved over the second straight tube section, and the first curved tube section may be sleeved over the second curved tube section, wherein each of the first and second curved tube sections has an angle of curvature, and wherein the angle of curvature of the first curved tube section is as same as the angle of curvature of the second curved tube section.

In another aspect, the present invention provides an implant delivery system comprising the catheter assembly as defined above and a delivery handle that is located at a proximal end of the catheter assembly. The delivery handle comprises an outer sheath tube control device, an inner tube control device and a guidewire tube fixation device. The outer sheath tube control device is coupled to the proximal end of the outer sheath tube and is configured to control the outer sheath tube to perform an axial movement. The inner tube control device is coupled to a proximal end of the inner tube and is configured to control the inner tube to perform an axial movement. The guidewire tube fixation device is coupled to the proximal end of the guidewire tube.

In a further aspect, the present invention provides an implant system comprising the catheter assembly as defined above, a delivery handle, a tapered head and a retaining head. The retaining head and the tapered head are sequentially disposed from proximal to distal at a distal end of the inner tube, the implant is crimped within the inner tube and is at least mostly confined between the tapered and retaining heads.

In yet a further aspect, the present invention provides a method for using the implant delivery system as defined above, which comprises:
choosing the first and second curved tube sections according to an anatomical structure of the heart of a patient, wherein the first and second curved tube sections have equal angles of curvature;
detachably attaching the proximal end of the first curved tube section to the distal end of the first straight tube section, and detachably attaching the proximal end of the second curved tube section to the distal end of the second straight tube section; and
elastically inserting the inner tube through a curved channel provided by the first and second curved tube sections.

Compared with the prior art, the present invention provides a catheter assembly, an implant system, an implant delivery system and a method of use thereof. In the catheter assembly, an inner tube is made of an elastic material, and an outer sheath tube and a guidewire tube are each made of a plastic material. A distal end portion of each of the outer sheath tube and the guidewire tube is shaped into a curved form to provide a curved channel for the inner tube. The inner tube is elastically inserted through the curved channel. This dispenses with the need for bending control from an outer sheath tube control device and an inner tube control device, reducing requirements and difficulties in designing them and thus resulting in cost savings. Moreover, it makes the implant delivery system more easily operable, safer and more reliable, with lower requirements on a surgical incision, and capable of delivering a stent through a curved access accurately to a designated site.

Further, in the implant delivery system of the present invention, a distal end of a first straight tube section is detachably attached to a proximal end of a first curved tube section, and a distal end of a second straight tube section is detachably attached to a proximal end of a second curved tube section. This enables the implant delivery system to accommodate various angles of curvature simply by appropriately designing the first and second curved tube sections, resulting in cost savings and increasing compatibility of the straight tube sections of the catheter assembly.

### BRIEF DESCRIPTION OF THE DRAWINGS

Fig. 1 is a schematic structural view of an implant delivery system according to an embodiment of the present invention.
Figs. 2a to 2b are schematic enlarged views of portion A of Fig. 1.
Figs. 3a to 3c shows how a first curved tube section is coupled to a first straight tube section according to embodiments of the present invention.
Figs. 4a to 4e are schematic structural views of an outer sheath tube according to embodiments of the present invention.
Figs. 5a to 5b are schematic structural views of a guidewire tube according to an embodiment of the present invention.
Fig. 6 is a schematic structural view of an implant delivery system deployed in the heart according to an embodiment of the present invention.

In these figures,
1, a catheter assembly; 110, an outer sheath tube; 111, a first straight tube section; 112, a first curved tube section; 113, an intermediate connecting core; 120, an inner tube; 121, a first inner sub-tube; 122, a second inner sub-tube; 130, a guidewire tube; 131, a second straight tube section; 132, a second curved tube section; 133, a third straight tube section; 140, a tapered head; 150, a retaining head;
2, a delivery handle; 21, an outer sheath tube control device; 22, an inner tube control device; 23, a guidewire tube fixation device; and
3, an implant.

### DETAILED DESCRIPTION

Catheter assemblies, implant systems, implant delivery systems and methods of use proposed in the present invention will be described in greater detail below. The invention will be described in greater detail below with reference to the accompanying drawings, which present preferred embodiments of the invention. It would be appreciated that those skilled in the art can make changes to the invention disclosed herein while still obtaining the beneficial results thereof. Therefore, the following description shall be construed as being intended to be widely known by those skilled in the art rather than as limiting the invention.

For the sake of clarity, not all features of actual implementations are described. In the following, description and details of well-known functions and structures are omitted to avoid unnecessarily obscuring the invention. It should be appreciated that in the development of any such actual implementation, numerous implementation-specific decisions must be made to achieve specific goals of the developers, such as compliance with system-related and business-related constrains, which may vary from one implementation to another. Moreover, it should be appreciated that such a development effort might be complex and time consuming, but would nevertheless be a routine undertaking for those of ordinary skill in the art.

Objects and features of the present invention will become more apparent upon reading the following detailed description of the present invention with reference to the accompanying drawings. Note that the figures are provided in a very simplified form not necessarily drawn to exact scale for the only purpose of facilitating easy and clear description of the embodiments disclosed herein. As used herein, the term "or" is generally employed in a sense including the meaning of "and/or", unless the context clearly dictates otherwise. As used herein, the terms "inner", "outer" and similar terms are merely illustrative and do not represent the only implementation possible. As used herein, the terms "proximal end" and "distal end" are employed to describe relative orientations, relative positions and directions between components of a medical device or actions thereof, as viewed by a surgeon operating the device. Without wishing to be limiting, a "proximal end" usually refers to an end closer to the operator, and a "distal end" usually refers to an end closer to the heart of a patient, during normal operation of the medical device.

In principle, the present invention provides a catheter assembly comprising an outer sheath tube, an inner tube and a guidewire tube, which are sequentially sleeved one over another from the outside inwards. The inner tube is made of an elastic material, and the outer sheath tube and the guidewire tube are each made of a plastic material. The distal end portions of the outer sheath tube and the guidewire tube are shaped into curve form to provide a curved channel for the inner tube, and the inner tube is elastically inserted through the curved channel.

An implant delivery system comprises a catheter assembly and a delivery handle disposed at a proximal end of the catheter assembly. The delivery handle comprises an outer sheath tube control device, an inner tube control device and a guidewire tube fixation device. The outer sheath tube control device is coupled to a proximal end of the outer sheath tube and is configured to control the outer sheath tube to perform an axial movement. The inner tube control device is coupled to a proximal end of the inner tube and is configured to control the inner tube to perform an axial movement. The guidewire tube fixation device is coupled to a proximal end of the guidewire tube.

An implant system comprises a catheter assembly, a delivery handle, a tapered head and a retaining head. The retaining and tapered heads are disposed, from proximal to distal, at a distal end of the inner tube. An implant is crimped within the inner tube and at least mostly restricted between the tapered and retaining heads.

A method for using the implant delivery system as defined above comprises:
choosing first and second curved tube sections according to an anatomical structure of the heart of a patient, wherein angles of curvature of the first and second curved tube sections are the same;
detachably attaching a proximal end of the first curved tube section to a distal end of a first straight tube section and detachably attaching a proximal end of the second curved tube section to a distal end of a second straight tube section; and
elastically inserting the inner tube through the curved channel provided by the first and second curved tube sections.

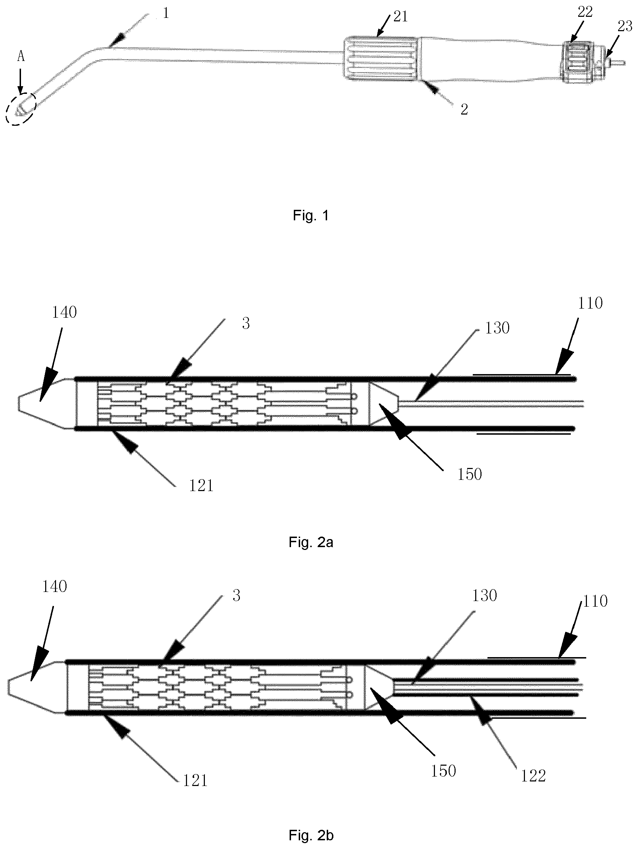

As shown in Fig. 1, embodiments of the present invention provide an implant delivery system comprising a catheter assembly 1 and a delivery handle 2, which are sequentially disposed from distal end to proximal end.

As shown in Figs. 2a to 2b, the catheter assembly 1 includes an outer sheath tube 110, an inner tube 120 and a guidewire tube 130, which are sequentiallysleeved one over another from the outside inwards. That is, the outer sheath tube 110 is sleeved over the inner tube 120, which is in turn sleeved over the guidewire tube 130. A guidewire is disposed within the guidewire tube 130.

The inner tube 120 is made of a soft material. That is, each inner sub-tube of the inner tube 120 is made of a soft material. The soft material may be a soft polymer material, such as Pebax, TPU, LDPE, NR or NBR, which has a Shore D hardness of 40-70 HD.

The inner tube 120 includes at least one inner sub-tube, such as one, two or more inner sub-tubes. That is, the inner tube 120 at least includes a first inner sub-tube 121. All the inner sub-tube(s) are sequentially sleeved one over another from the inside outwards, and the first inner sub-tube 121 is located at the outermost layer of the inner tube.

The delivery handle 2 includes an outer sheath tube control device 21, an inner tube control device 22 and a guidewire tube fixation device 23. The outer sheath tube control device 21 is coupled to a proximal end of the outer sheath tube 110 and is configured to control the outer sheath tube 110 to perform an axial movement. The inner tube control device 22 is coupled to a proximal end of the inner tube 120 and is configured to control the inner tube 120 to perform an axial movement. Specifically, the inner tube control device 22 includes at least one inner sub-tube control device, and the number of inner sub-tube control device is equal to that of inner sub-tube(s). Each inner sub-tube control device is coupled to a proximal end of an inner sub-tube, and is configured to control axial movement of a respective one of the inner sub-tube(s). The guidewire tube fixation device 23 is coupled to and fixes a proximal end of the guidewire tube 130.

The outer sheath tube 110 is made of a hard material, such as a hard polymer or metal material. The polymer material may be, for example, PA, PI, PEEK or the like, and may have a Shore D hardness of 70 HD to 90 HD. The metal material may be 304 stainless steel, 316 stainless steel or the like and may have a Rockwell hardness of 20-50 HRC.

Curvature required for an implant delivery system to be delivered through a curved access is typically characterized with an angle of curvature A and a radius of curvature R. An access for delivery of a prosthetic valve is usually not straight, a certain angle is required, commonly known as an angle of curvature. Moreover, an access for delivery of a prosthetic valve is established within the heart, and due to space restrictions of the atrial and ventricular chambers, a system used to deliver the implant is required to provide bending control at various radii, commonly known as radii of curvature. In order to enable the implant delivery system to be curved to curvature required by an intended access for delivery, the outer sheath tube 110 includes a first straight tube section 111 and a first curved tube section 112, which are coupled to each other axially. The first straight tube section 111 is located at a proximal end portion of the outer sheath tube 110, and the first curved tube section is located at a distal end portion of the outer sheath tube 110. The first straight tube section 111 comprises a straight axis, while the first curved tube section 112 comprises a curved axis, for example, an arc-shaped axis. By virtue of the curvature defined by the curved axis of the first curved tube section 112 and the non-deformable material of its own, the outer sheath tube 110 can be advanced through an access with an angle of curvature without requiring bending control of the outer sheath tube control device 21. This reduces requirements and difficulties in designing the outer sheath tube control device 21, resulting in cost savings.

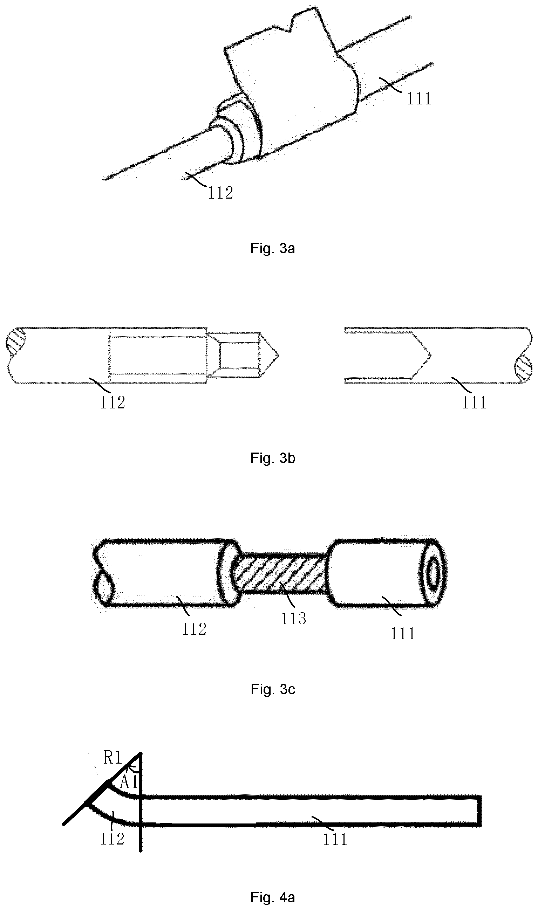

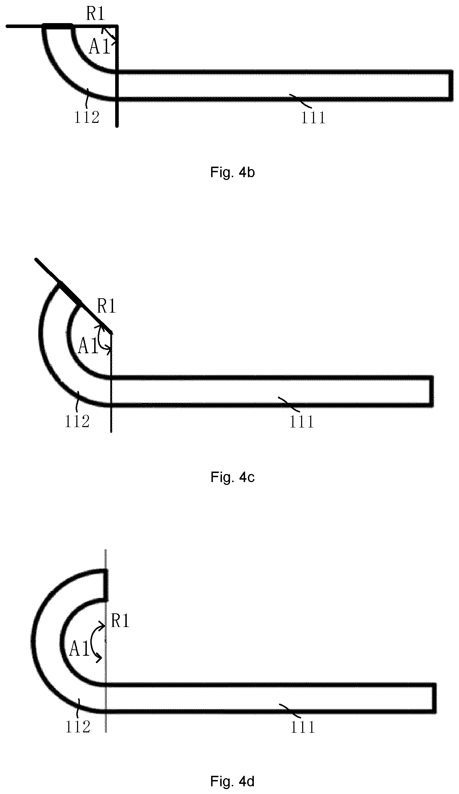

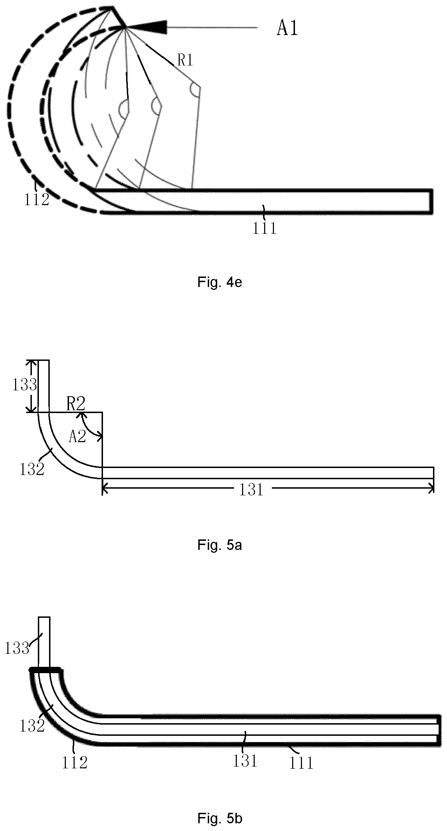

Depending on a radius of curvature required by bending control of the implant delivery system, the first curved tube section 112 may be designed to have various radii of curvature R1. Moreover, depending on an intended access for the implant delivery system, various angles of curvature A may be designed. Specifically, at a given radius of curvature R1, the angle of curvature A1 may be variously adjusted. The angle of curvature A1 is greater than 0° and may be a commonly-used value such as 30°, 45° (Fig. 4a), 60°, 90° (Fig. 4b), 120°, 135° (Fig. 4c), 180° (Fig. 4d), 270° or 360°. Of course, it may also be any other value greater than 0°, such as 10°, 15°, 20°, 50° or 144°. As shown in Fig. 4e, at a given angle of curvature A1, the radius of curvature R1 may be variously adjusted.

A proximal end of the first curved tube section 112 is detachably coupled to a distal end of the first straight tube section 111. In this way, it is possible for the implant delivery system to accommodate various angles of curvature simply by appropriately designing the first curved tube section. Specifically, as shown in Fig. 3a, the proximal end of the first curved tube section 112 may be boned to the distal end of the first straight tube section 111 by an adhesive applied to the exterior of the outer sheath tube 110. As shown in Fig. 3b, the proximal end of the first curved tube section 112 may be provided with an external thread, and the distal end of the first straight tube section 111 may be provided with an internal thread engageable with the external thread. In this case, the proximal end of the first curved tube section 112 may be threadedly coupled to the distal end of the first straight tube section 111. Alternatively, the proximal end of the first curved tube section 112 may be provided with an internal thread, and the distal end of the first straight tube section 111 may be provided with an external thread engageable with the internal thread. In this case, the proximal end of the first curved tube section 112 may also be threadedly coupled to the distal end of the first straight tube section 111. As shown in Fig. 3c, the outer sheath tube 110 may further include a tubular intermediate connecting core 113 having the same inner diameter as the outer sheath tube 110. The intermediate connecting core 113 may have external threads on its opposite end portions, and the proximal end of the first curved tube section 112 and the distal end of the first straight tube section 111 may have respective internal threads engageable with the external threads. In this case, the proximal end of the first curved tube section 112 and the distal end of the first straight tube section 111 may be threadedly coupled to the opposite end portions of the intermediate connecting core 113. Alternatively, the intermediate connecting core 113 may have an inner diameter equal to an outer diameter of the outer sheath tube 110 and internal threads on its opposite end portions, and the proximal end of the first curved tube section 112 and the distal end of the first straight tube section 111 may have respective external threads engageable with the internal threads. In this case, the proximal end of the first curved tube section 112 and the distal end of the first straight tube section 111 may also be threadedly coupled to the opposite end portions of the intermediate connecting core 113.

In another embodiment, the outer sheath tube 110 may further include a distal straight tube section, which has a straight axis and is coupled to at its proximal end to a distal end of the first curved tube section 112.

The guidewire tube 130 is made of a hard material, such as a hard polymer or metal material. The polymer material may be, for example, PA, PI, PEEK or the like, and may have a Shore D hardness of 70 HD to 90 HD. The metal material may be 304 stainless steel, 316 stainless steel or the like and may have a Rockwell hardness of 20-50 HRC.

As shown in Figs. 5a to 5b, the guidewire tube 130 includes a second straight tube section 131, a second curved tube section 132 and a third straight tube section 133, which are sequentially joined together along an axis of the guidewire tube 130. The second straight tube section 131 is located at a proximal end portion of the guidewire tube 130, and the third straight tube section 133 is located at a distal end portion of the guidewire tube 130. The first straight tube section 111 is sleeved over the second straight tube section 131, with the third straight tube section 133 protruding out of the outer sheath tube 110 from the distal end thereof. That is, the third straight tube section 133 protrudes out of the distal end of the first curved tube section 112. The first curved tube section 112 is sleeved over the second curved tube section 132. Both the second straight tube section 131 and the third straight tube section 133 comprise straight axes. The second curved tube section 132 comprises a curved axis, in particular, for example, an arc-shape axis. An angle of curvature A2 of the second curved tube section 132 is equal to the angle of curvature A1 of the first curved tube section 112. In this way, the first curved tube section 112 and the second curved tube section 132 are arranged parallel to each other, allowing smooth passage of the inner tube 120 between the first curved tube section 112 and the second curved tube section 132. Since the outer sheath tube 110 and the guidewire tube 130 have the same angle of curvature and are both made of non-deformable hard materials, the curved shape (curved shape of the first and second curved tube sections) together formed by the guidewire tube 130 and the outer sheath tube 110 along axis thereof provides a curved channel. The inner tube 120 located between the outer sheath tube 110 and the guidewire tube 130 made of a softer material has a certain degree of deformability, enabling the inner tube 120 to perform the bending movement so as to ensure the inner tube 120 can smoothly pass through the curved channel formed by the first and second curved tube sections. Meanwhile, the inner tube 120 is of sufficient stiffness to maintain the crimped implant, allowing loading and release of the implant. Further, the curved channel provided by the outer sheath tube 110 and the guidewire tube 130 allows the softer inner tube 120 to pass through a curved access without requiring separate bending control. This reduces requirements and difficulties in designing the outer sheath tube control device 21 and the inner tube control device 22, resulting in cost savings. Moreover, it makes the implant delivery system more easily operable, safer and more reliable, with lower requirements on a surgical incision, and capable of delivering a stent through a curved access accurately to a designated site.

A proximal end of the second curved tube section 132 is detachably coupled to a distal end of the second straight tube section 131. Specifically, this may be accomplished by, for example, adhesive bonding (e.g., as shown in Fig. 3a), direct threaded coupling (e.g., as shown in Fig. 3b) or threaded coupling using an intermediate connecting core 113 (e.g., as shown in Fig. 3c). The second curved tube section 132 and the third straight tube section 133 may be integrally formed as one piece, or detachably coupled to each other (in particular, using any of the coupling methods shown in Figs. 3a to 3c).

Referring to Fig. 1, embodiments of the present invention also provide an implant system including the catheter assembly as defined above and a delivery handle. A distal end portion of the catheter assembly 1 further includes a tapered head 140 and a retaining head 150. The retaining head 150 is disposed within a distal end portion of the first inner sub-tube 121 so as to be limited in six degrees of freedom and is configured to retain an implant 3. The tapered head 140 is detachably disposed at the distal end of the first inner sub-tube 121. The implant 3 is confined at the proximal end of the retaining head 150. The implant 3 is, for example, a valve stent. The number of inner sub-tubes in the inner tube may be determined as required by the implant 3.

As shown in Fig. 2a, the implant 3 may include only a body, and the inner tube 120 may include only the first inner sub-tube 121. In this case, the tapered head 140 is disposed in the distal end portion of the first inner sub-tube 121, and the implant 3 is confined between the retaining head 150 and the tapered head 140.

As shown in Fig. 2b, the implant 3 may include a distal body and a proximal flange, and the inner tube 120 may include the first inner sub-tube 121 and a second inner sub-tube 122. In this case, the first inner sub-tube 121 is sleeved over the second inner sub-tube 122, and a distal portion of the first inner sub-tube 121 extends out of distal end of the second inner sub-tube 122. Additionally, the distal end of the second inner sub-tube 122 is coupled to the retaining head 150. The body is confined between the retaining head 150 and the tapered head 140, and the flange is secured at the distal end of the second inner sub-tube 122.

A method for using the implant delivery system as defined above is described below.

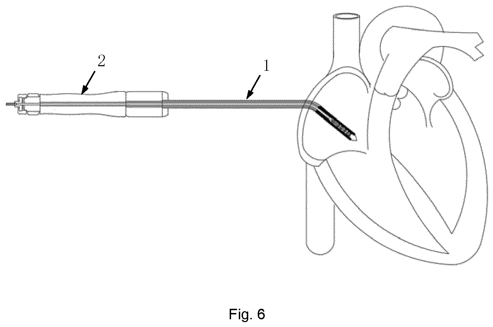

In the case of the inner tube including only the first inner sub-tube 121, at first, an anatomical structure of the heart of a patient is preoperatively mapped using ultrasound waves or the like, and a required angle of curvature is determined. According to the required angle of curvature, the first curved tube section 112 and the second curved tube section 132 are chosen to have the same angle of curvature as the desired one or a commonly-used angle of curvature (e.g., 30°, 45°, 60°, 90° or the like) close to the desired one (of course, they may also be customized exactly to the desired angle of curvature). The first curved tube section 112 is deployed at the distal end of the first straight tube section 111, and the second curved tube section 132 is deployed at the distal end of the second straight tube section 131. The third straight tube section 133 is disposed at a distal end of the second curved tube section 132, and the first inner sub-tube 121 is inserted between the outer sheath tube 110 and the guidewire tube 130. Subsequently, a control device for the first inner sub-tube 121 is manipulated to deploy an implant at a distal end of the implant delivery system. That is, the implant is crimped within the distal end portion of the first inner sub-tube and confined between the retaining head 150 and the tapered head 140. This completes the deployment of the implant in the implant delivery system. After that, as shown in Fig. 6, the curved implant delivery system is delivered to a designated site in the heart, and the control device for the first inner sub-tube 121 is then manipulated to enable safe release of the implant.

In the case of the inner tube 120 including the first inner sub-tube 121 and the second inner sub-tube 122, at first, an anatomical structure of the heart of a patient is preoperatively mapped using ultrasound waves or the like, and a required angle of curvature is determined. According to the required angle of curvature, the first curved tube section 112 and the second curved tube section 132 are chosen to have the same angle of curvature as the desired one or a commonly-used angle of curvature (e.g., 30°, 45°, 60°, 90° or the like) close to the desired one. The first curved tube section 112 is deployed at the distal end of the first straight tube section 111, and the second curved tube section 132 is deployed at the distal end of the second straight tube section 131. The first inner sub-tube 121 and the second inner sub-tube 122 are inserted between the outer sheath tube 110 and the guidewire tube 130, and the first inner sub-tube 121 is disposed over the second inner sub-tube 122. Subsequently, control device(s) for the first inner sub-tube 121 and the second inner sub-tube 122 are manipulated to deploy an implant at a distal end of the implant delivery system so that a body of the implant is crimped within a distal end portion of the second inner sub-tube 122 and confined between the retaining head 150 and the tapered head 140 and that a flange of the implant is secured at the distal end of the first inner sub-tube 121. This completes the deployment of the implant in the implant delivery system. After that, as shown in Fig. 6, the curved implant delivery system is delivered to a designated site in the heart, and the control means for the first inner sub-tube 121 and the second inner sub-tube 122 are manipulated to enable safe release of the implant.

In summary, the present invention provides a catheter assembly, an implant system, an implant delivery system and a method of use thereof. The delivery system has a fixed angle of curvature and is used to implant a prosthetic valve. The implant delivery system of the present invention does not pose high requirements on a surgical incision, and its curvature enables it to deliver a stent through a curved access accurately to a designated site. In addition, according to the present invention, each of a guidewire tube and an outer sheath tube for providing the curvature is divided into a straight section and a curved section, which are coupled to each other detachably. This not only results in significant cost savings, but also increases compatibility of the straight sections. According to the present invention, bending control of the delivery system can be achieved with a simple structural design and a relatively small number of components. Further, it provides a range of advantages including easy operation, high safety and high reliability.

It is noted that, as used herein, the terms "first" and "second" are only meant to distinguish various components, elements, steps, etc. from each other rather than indicate logical or sequential orderings thereof, unless otherwise indicated or specified.

It is understood that while the invention has been described above with reference to preferred embodiments thereof, it is not limited to these embodiments. In light of the above teachings, any person familiar with the art may make many possible modifications and variations to the disclosed embodiments or adapt them into equivalent embodiments, without departing from the scope of the invention. Accordingly, it is intended that any and all simple variations, equivalent changes and modifications made to the foregoing embodiments based on the substantive disclosure of the invention without departing from the scope thereof fall within this scope.

## Claims

1. A catheter assembly, comprising an outer sheath tube, an inner tube and a guidewire tube that are sequentially sleeved one over another from outside inwards, wherein: the inner tube is made of an elastic material; and each of the outer sheath tube and the guidewire tube is made of a plastic material, wherein a distal end of each of the outer sheath tube and the guidewire tube is shaped into a curved form to provide a curved channel for the inner tube, and wherein the inner tube is elastically inserted through the curved channel.

2. The catheter assembly of claim 1, wherein the outer sheath tube is made of a polymer material with a Shore D hardness of 70-90 HD, or
wherein the outer sheath tube is made of a metal material with a Rockwell hardness of 20-50 HRC.

3. The catheter assembly of claim 1, wherein the guidewire tube is made of a polymer material with a Shore D hardness of 70-90 HD, or
wherein the guidewire tube is made of a metal material with a Rockwell hardness of 20-50 HRC.

4. The catheter assembly of claim 2 or 3, wherein the polymer material comprises PA, PI or PEEK, and wherein the metal material comprises 304 stainless steel or 316 stainless steel.

5. The catheter assembly of claim 1, wherein the inner tube is made of Pebax, TPU, LDPE, NR or NBR and has a Shore D hardness of 40-70 HD.

6. The catheter assembly of claim 1, wherein the outer sheath tube comprises, disposed along an axis thereof, a first straight tube section and a first curved tube section, wherein the first straight tube section is located at a proximal end of the outer sheath tube, wherein the first curved tube section is located at the distal end of the outer sheath tube, and wherein a distal end of the first straight tube section is detachably coupled to a proximal end of the first curved tube section.

7. The catheter assembly of claim 1, wherein the guidewire tube comprises, sequentially disposed along an axis thereof, a second straight tube section, a second curved tube section and a third straight tube section, wherein the second straight tube section is located at a proximal end of the guidewire tube, wherein the third straight tube section is located at the distal end of the guidewire tube, and wherein a distal end of the second straight tube section is detachably coupled to a proximal end of the second curved tube section.

8. The catheter assembly of claim 7, wherein: the first straight tube section is sleeved over the second straight tube section; and the first curved tube section is sleeved over the second curved tube section, wherein each of the first and second curved tube sections has an angle of curvature, and wherein the angle of curvature of the first curved tube section is equal to the angle of curvature of the second curved tube section.

9. An implant delivery system, comprising the catheter assembly of any one of claims 1 to 8 and a delivery handle that is located at a proximal end of the catheter assembly, wherein the delivery handle comprises an outer sheath tube control device, an inner tube control device and a guidewire tube fixation device, wherein the outer sheath tube control device is coupled to a proximal end of the outer sheath tube and is configured to control the outer sheath tube to perform an axial movement, wherein the inner tube control device coupled to a proximal end of the inner tube and is configured to control the inner tube to perform an axial movement, and wherein the guidewire tube fixation device is coupled to a proximal end of the guidewire tube.

10. An implant system, comprising the catheter assembly of any one of claims 1 to 8, a delivery handle, a tapered head and a retaining head, wherein the retaining head and the tapered head are sequentially disposed from proximal to distal at a distal end of the inner tube, wherein an implant is crimped within the inner tube and is at least mostly confined between the tapered and retaining heads.

11. A method for using the implant delivery system of claim 9, comprising:
choosing a first curved tube section and a second curved tube section according to an anatomical structure of a heart of a patient, wherein the first and second curved tube sections have equal angles of curvature;
detachably attaching a proximal end of the first curved tube section to a distal end of the first straight tube section, and detachably attaching a proximal end of the second curved tube section to a distal end of the second straight tube section; and
elastically inserting the inner tube through a curved channel provided by the first and second curved tube sections.
